**Europäisches Patentamt**

**European Patent Office**

**Office européen des brevets**

(19)

(11) Publication number:

**0 078 589**
**B1**

## EUROPEAN PATENT SPECIFICATION

(12)

(45) Date of publication of patent specification: **10.09.86**

(21) Application number: **82201585.5**

(22) Date of filing: **04.06.82**

(60) Publication number of the earlier application in accordance with Art. 76 EPC: **0 068 658**

(51) Int. Cl.⁴: **F 16 B 2/14**

(54) **Demountable joint.**

(30) Priority: **09.06.81 GB 8117574**

(43) Date of publication of application:
**11.05.83 Bulletin 83/19**

(45) Publication of the grant of the patent:
**10.09.86 Bulletin 86/37**

(84) Designated Contracting States:
**DE FR**

(56) References cited:
**FR-A-2 094 227**
**US-A-3 142 881**

**O.Richter und R.v.Voss ,Bauelemente der Feinmechanik,Neunte Auflage,1964,pages 275 and 276**
**Augustus D.Hargan,Handbook of fastening and joining of metal parts,first edition,1956,page 539**

(73) Proprietor: **J.E. HANGER & COMPANY LIMITED**
**Roehampton Lane**
**Roehampton London SW15 5PL (GB)**

(72) Inventor: **May, Denis Ronald William**
**c/o J. E. Hanger & Company Ltd. Roehampton Lane**
**Roehampton London, SW15 5PL (GB)**

(74) Representative: **Cole, Paul Gilbert et al**
**Hughes Clark Andrews & Byrne 63 Lincoln's Inn Fields**
**London WC2A 3JU (GB)**

EP 0 078 589 B1

Courier Press, Leamington Spa, England.

## Description

The present invention relates to a demountable joint by which a tube may be clamped to a socket.

There is described in O. Richter and R. V. Voss "Bauelemente der Feinmechanik" VEB Verlag Technik Berlin, 9th Edition at page 275 with reference to Figure 42, 45, a clamp comprising a sleeve having a main bore, a cylindrical member in the main bore, an auxiliary bore in the sleeve directed perpendicular to the axis of the main bore and intersecting it over a small arc, first and second collets that each fit in the auxiliary bore and have at their respective inner ends part cylindrical bearing faces that conform to the curvature of the cylindrical member, and clamping bolt means that urges the first and second collets together so that they press on the tubular member and hold the sleeve in axial position. The cylindrical member in "Bauelemente der Feinmechanik" is a solid rod, and in Laughner and Hargan, "Handbook of Fastening and Joining of Metal Parts", McGraw Hill, 1956, a similar clamp is shown in use with a thick walled tube. In each case the clamp can slide freely along the whole length of the cylindrical member until the clamping bolt is tightened to immobilise it.

This invention is concerned with the problem of effectively clamping an end of a tube such as the shin pylon tube of an artificial leg into a socket to hold the tube permanently in place without drilling or slitting the tube which can weaken it and reduce fatigue life.

The invention provides a demountable joint comprising a main bore, a cylindrical member in the main bore, and auxiliary bore directed perpendicular to the axis of the main bore and intersecting it over a small arc, first and second collets that each fit in the auxiliary bore and have at their respective inner ends part cylindrical bearing faces conforming to the curvature of the cylindrical member and clamping bolt means that urges the first and second collets together so that they press on the cylindrical member, characterised in that the cylindrical member is a tube that is fixed endwise into a socket and is protected from deforming under the pressure exerted by the collets by means of a reinforcing insert that fits into an end of the tube with a locating flange seating on the end of the tube and with a disc or annulus positioned so that when the end of the tube is fully home in the socket the disc or annulus registers with the collets to resist compressive loads on the tube.

An embodiment of the invention will now be illustrated in the accompanying drawings in which:

Figure 1 is a view in vertical section of a demountable fastening for fixing the shin pylon tube into the socket of the shin member in an artificial leg; and

Figure 2 is a view of the joint in horizontal section on the line A—A of Figure 1.

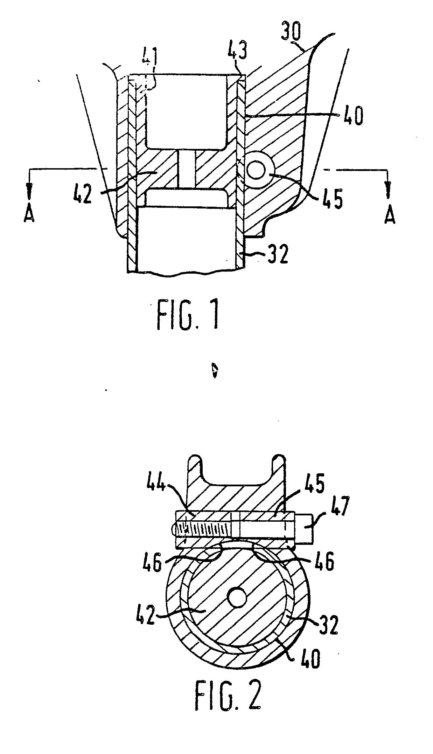

In Figures 1 and 2, the upper end of a shin pylon tube 32 is a push fit in a socket 40 defined by a vertical bore in the lower face of knee housing 30. A reinforcing insert 41 is a push fit in the top of tube 32. It is formed towards its lower ends with a horizontally directed reinforcing annulus 42 which is capable of withstanding high compressive loads and at its top with an outwardly directed locating flange 43 which acts as an abutment for the top end of the tube 32. As more clearly seen in Figure 2 the socket 40 is formed with a horizontal bore that intersects the vertical bore in which tube 32 fits over a small arcuate portion thereof. A pair of collets 44, 45 fit into the horizontal bore, one of them 45 having a plain through-hole and the other 44 having a threaded through hole. Each collet has at its inner end a part cylindrical bearing surface 46 whose axis is normal to that of the collet and which conforms to the curvature of the outer surface of the tube 32 which can only be inserted fully home in the socket 40 when the bearing surfaces 46 are appropriately positioned. It will be noted that in the assembled joint the horizontal bore registers with the reinforcing annulus 42 of the insert 41. A clamping bolt 47 is inserted through collet 45 into collet 44 in which it is threadedly located. As the bolt 47 is tightened the collets 44, 45 are urged together and their surfaces 46 press on the tube 32 to retain it in the socket 40.

A knee joint for artificial limbs is disclosed in EP—A—68658, published 05.01.83.

## Claims

1. A demountable joint comprising a main bore, a cylindrical member (32) in the main bore, an auxiliary bore directed perpendicular to the axis of the main bore and intersecting it over a small arc, first and second collets (44, 45) that each fit in the auxiliary bore and have at their respective inner ends part cylindrical bearing faces (46) conforming to the curvature of the cylindrical member (32), and clamping bolt means (47) that urges the first and second collets (44, 45) together so that they press on the cylindrical member (32), characterised in that the cylindrical member (32) is a tube that is fixed endwise into a socket (40) and is protected from deforming under the pressure exerted by the collets (44, 45) by means of a reinforcing insert (41) that fits into an end of the tube (32) with a locating flange (43) seating on the end of the tube (32) and with a disc or annulus (42) positioned so that when the end of the tube (32) is fully home in the socket (40) the disc or annulus (42) registers with the collets (44, 45) to resist compressive loads on the tube (32).

2. A joint according to Claim 1, wherein the cylindrical member (32) is a shin pylon tube of an artificial leg.

## Patentansprüche

1. Abnehmbarer Verbinder mit einer Hauptbohrung, einem zylindrischen Glied (32) in der Hauptbohrung, einer Hilfsbohrung, die senkrecht zu der Achse der Hauptbohrung gerichtet ist und diese in einem kleinen Bogenbereich durchschneidet, und einer ersten und zweiten Klemmhülse (44, 45), die jede in die Hilfsbohrung paßt und an ihren jeweiligen inneren Enden teilzylindrische Tragflächen (46) aufweist, die mit dem Kurvenverlauf des zylindrischen Gliedes (32) übereinstimmen, und einem Randbolzenmittel (47), das die erste und die zweite Klemmhülse (44, 45) zusammentreibt, so daß sie auf das zylindrische Glied (32) pressen, dadurch gekennzeichnet, daß das zylindrische Glied (32) ein Rohr ist, das aufrecht in einer Fassung (40) fixiert ist und vor einer Verformung unter dem Druck der von den Klemmhülsen (44, 45) ausgeübt wird durch das Mittel eines Versteifungseinsatzes (41) geschützt ist, der in ein Ende des Rohres (32) paßt, mit einem Paßflansch (43), der auf dem Ende des Rohres (32) aufliegt und mit einer Scheibe oder Ringspule (42), die so angeordnet ist, daß dann wenn das Ende des Rohres (32) vollkommen in die Fassung (40) eingeführt ist, die Scheibe oder Ringspule (42) mit den Klemmhülsen (44, 45) zusammenwirkt, um gegen Druckkräfte auf das Rohr (32) beständig zu sein.

2. Verbinder nach Anspruch 1, worin das zylindrische Glied (32) ein Schienbeinpilonrohr eines künstliches Beines ist.

**Revendications**

1. Joint démontable comprenant un alésage principal, un élément cylindrique (32) placé dans l'alésage principal, un trou auxiliaire disposé perpendiculairement à l'axe de l'alésage principal et le coupant sur un petit arc, de premier et second collet (44, 45) s'emboîtant chacun dans le trou auxiliaire et comportant à leurs extrémités intérieures respectives des faces de support cylindriques (46) se conformant à la courbure de l'élément cylindrique (32), et des moyens de boulon de blocage (47) pousant le premier et second collet (44, 45) l'une contre l'autre de façon qu'ils viennent s'appuyer sur l'élément cylindrique (32), joint démontable caractérisé en ce que l'élément cylindrique (32) est un tube fixé par son extrémité dans un logement (40) et protégé contre les déformations sous la pression exercée par les collets (44, 45), par une pièce intérieure de renforcement (41) s'emboîtant dans une extrémité du tube (32), avec un rebord de mise en place (43) venant s'appuyer sur l'extrémité du tube (32), et avec une disque ou partie annulaire (42) venant se placer de façon que lorsque l'extrémité du tube (32) est complètement logée dans le logement (40), le disque ou partie annulaire (42) vienne coïncider avec les collets (44, 45) pour résister aux efforts de compression appliqués au tube (32).

2. Joint selon la revendication 1, caractérisé en ce que l'élément cylindrique (32) est un tube de tibia de jambe artificielle.

FIG. 1

FIG. 2

1